(19) Europäisches Patentamt
European Patent Office
Office européen des brevets

(11) Numéro de publication : **0 349 406 B1**

(12) # FASCICULE DE BREVET EUROPEEN

(45) Date de publication du fascicule du brevet : **18.03.92 Bulletin 92/12**

(51) Int. Cl.$^5$ : **C07C 59/153,** C07C 51/235, C07C 51/27

(21) Numéro de dépôt : **89401809.2**

(22) Date de dépôt : **26.06.89**

(54) **Procédé de fabrication industrielle de solutions aqueuses d'acide glyoxylique.**

(30) Priorité : **01.07.88 FR 8808957**

(43) Date de publication de la demande : **03.01.90 Bulletin 90/01**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**GB-A- 2 001 621**
**US-A- 2 298 387**

(73) Titulaire : **SOCIETE FRANCAISE HOECHST**
**TOUR ROUSSEL HOECHST 1 Terrasse Bellini**
**F-92800 Puteaux (FR)**

(72) Inventeur : **Schouteeten, Alain**
**17 rue de Normandie**
**F-95460 Ezanville (FR)**
Inventeur : **Christidis, Yani**
**53 Boulevard de la Villette**
**F-75019 Paris (FR)**

(74) Mandataire : **Santarelli, Marc**
**Cabinet Rinuy et Santarelli 14, avenue de la Grande Armée**
**F-75017 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé d'obtention industrielle de solutions aqueuses d'acide glyoxylique.

Il est connu d'oxyder des solutions aqueuses de glyoxal en des solutions aqueuses d'acide glyoxylique par de l'acide nitrique, en présence, éventuellement soit d'un acide minéral fort tel que l'acide chlorhydrique, soit d'acide oxalique, soit d'un sel soluble de cobalt, soit enfin d'oxygène moléculaire (H. Debus, Annalen , 1857 , 102, 28 ; brevets français N° 1.326.605, 2.372.141 et 2.516.506 ; brevets de la République Fédérale d'Allemagne N° 932.369, 933.987, 1.002.309 et 31.32.006 ; demandes de brevet japonais N° 73-103.517, 76-29441, 76-80821, 77-105121 et 80-129440).

Ces procédés fournissent des solutions aqueuses d'acide glyoxylique contenant, outre du glyoxal et de l'acide nitrique non transformés, des quantités relativement importantes d'acide oxalique, et ils libèrent de grandes quantités d'oxydes d'azote qui exigent de coûteux moyens d'absorption pour éviter leur rejet dans l'atmosphère.

La présence dans des solutions aqueuses d'acide glyoxylique de glyoxal et d'acide nitrique est particulièrement gênante du fait de leur réactivité vis-à-vis de l'acide glyoxylique, aussi leur élimination doit elle être effectuée rapidement, de manière à obtenir une concentration résiduelle en glyoxal inférieure à 2 % en proportions molaires par rapport à l'acide glyoxylique et une concentration résiduelle en acide nitrique inférieure à 0,05 % en proportions molaires par rapport à l'acide glyoxylique. L'élimination du glyoxal des solutions aqueuses d'acide glyoxylique est particulièrement délicate et onéreuse, du fait que ces deux produits possèdent au moins une fonction aldéhyde.

Récemment, il a été proposé, dans le brevet français N° 2.516.506, un procédé d'oxydation de solutions aqueuses d'acide glyoxylique par un agent oxydant, obtenu à partir d'acide nitrique et d'un acide fort non oxydant présent à une concentration pondérale de 6 à 40% dans le mélange réactionnel. Malgré son intérêt pour les faibles concentrations résiduelles en acide nitrique présentes dans les solutions aqueuses d'acide glyoxylique obtenues, ce procédé ne répond toutefois pas aux exigences du marché en raison des concentrations élevées en glyoxal non transformé.

Or la Demanderesse a découvert un nouveau procédé de fabrication industrielle de solutions aqueuses d'acide glyoxylique obviant à ces inconvénients.

Selon le procédé de la présente invention, on prépare des solutions aqueuses d'acide glyoxylique en faisant réagir à un pH inférieur à 1, une solution aqueuse de glyoxal avec de l'oxygène, en présence de quantités catalytiques de monooxyde d'azote.

Comme solutions aqueuses de glyoxal, on peut utiliser les solutions aqueuses de glyoxal disponibles dans l'industrie et contenant pondéralement de 5 à 40 % de glyoxal, avantageusement celles contenant de 10 à 30 % de glyoxal et, préférentiellement, une solution aqueuse de glyoxal à 17 ± 3 % en poids.

Le procédé selon l'invention est mis en oeuvre à un pH inférieur à 1. Pour ce faire, on acidifie la solution aqueuse de glyoxal de départ avec un acide minéral fort tel que l'acide chlorhydrique, l'acide sulfurique ou leurs mélanges en proportions variables. Avantageusement, on acidifie les solutions aqueuses de glyoxal avec 2 moles d'acide chlorhydrique par litre de solution.

Le procédé selon l'invention est réalisé sous un barbotage d'oxygène moléculaire (dioxygène), à une pression comprise entre $10^5$ et 8 x $10^5$ Pa, avantageusement à une pression de 4 x $10^5$ Pa. On met en oeuvre le procédé à la température la plus souhaitable, généralement à une température comprise entre 35 et 75°C, avantageusement entre 40 et 70°C.

Le catalyseur d'oxydation est le monooxyde d'azote, NO. On utilise habituellement entre environ 75 et 150 mmoles de monooxyde d'azote par mole de glyoxal mise en oeuvre. En fin de réaction, ce catalyseur est aisément éliminé du milieu réactionnel par des moyens connus en eux-mêmes. En présence d'oxygène, ce catalyseur s'oxyde spontanément en dioxyde d'azote, $NO_2$, ou en son dimère, le tétraoxyde d'azote, $N_2O_4$. Au contact du glyoxal, le dioxyde d'azote se réduit en monooxyde d'azote et oxyde le glyoxal en acide glyoxylique.

Le schéma réactionnel peut s'écrire comme suit :

$$2NO + O_2 \longrightarrow 2NO_2$$

$$2NO_2 \rightleftarrows N_2O_4$$

$$2OHC-CHO + 2NO_2 \longrightarrow 2OHC-COOH + 2NO$$

L'équation réactionnelle globale est donc la suivante :

$$_2OHC - CHO + O_2 \rightarrow {}_2OHC - COOH$$

Ces réactions sont rapides et exothermiques. En conséquence, la température de la réaction doit être maintenue à la valeur souhaitée par un moyen approprié d'élimination de la chaleur.

Théoriquement, il n'y a pas de consommation de monooxyde d'azote. Cependant, en raison de l'imperfection de l'appareillage et/ou de quelques réactions secondaires, on constate parfois une légère consommation de monooxyde d'azote, inférieure toutefois à 80 mmoles par mole de glyoxal mis en oeuvre, que l'on compense, lorsque le procédé est réalisé en continu, par l'addition de la quantité correspondante de monooxyde d'azote frais.

Un des intérêts du procédé de la présente invention est qu'il s'effectue sans dilution de la solution aqueuse de glyoxal mise en oeuvre.

Le procédé selon la présente invention est sélectif. Ainsi, pour un taux de transformation de 99 %, on note une sélectivité de 80 ± 5 %, c'est-à-dire que l'on recueille en fin de réaction 80 ± 5 % du glyoxal engagé sous forme d'acide glyoxylique. L'oxydation du glyoxal soit en acide oxalique, soit en dioxyde de carbone, est au maximum de 25 % pour l'acide oxalique et de 1 % pour le dioxyde de carbone, exprimés en proportions molaires par rapport à l'acide glyoxylique obtenu.

En marche continue, la réaction d'oxydation peut être suivie par l'analyse, par des moyens connus en eux-mêmes, des gaz exhalés, notamment de l'oxygène, du dioxyde de carbone, des oxydes d'azote et elle peut être réglée par ajustement de l'un ou de plusieurs de ses paramètres réactionnels tels que la température, la pression, le débit d'introduction de l'oxygène, le débit d'alimentation en glyoxal.

Le procédé selon la présente invention permet d'obtenir des solutions aqueuses d'acide glyoxylique quasiment exemptes d'acide nitrique, et contenant, en proportions molaires par rapport à l'acide glyoxylique présent, moins de 2,5 % de glyoxal. Par ailleurs, il libère dans l'environnement très peu ou pas d'oxydes d'azote.

En fin de réaction, l'acide oxalique éventuellement présent dans les solutions aqueuses d'acide glyoxylique obtenues est éliminé par des moyens connus en eux-mêmes. Avantageusement, on le récupère par cristallisation de son hydrate à deux molécules d'eau et les dernières traces sont éliminées soit par électrodialyse, soit par traitement avec une résine échangeuse d'ions, simultanément, si désiré, avec l'élimination du ou des acides minéraux utilisés pour acidifier à pH inférieur à 1, la solution aqueuse de glyoxal de départ. En effet, le ou les acides minéraux introduits dans la solution de départ de glyoxal peuvent être récupérés en fin de réaction en soumettant les solutions aqueuses d'acide glyoxylique obtenues soit à un traitement par des résines échangeuses d'ions convenablement choisies, soit à un traitement d'électrodialyse bien connu par ailleurs. Pour obtenir une solution aqueuse ayant une concentration en acide glyoxylique supérieure à celle qui peut être obtenue directement par le procédé de la présente invention, la solution aqueuse d'acide glyoxylique peut éventuellement être concentrée par des techniques connues.

Avantageusement, le procédé de la présente invention est réalisé en continu soit dans une série de réacteurs agités, disposés en cascade, équipés d'un système adéquat de recyclage des gaz, soit dans une colonne liquide-gaz. L'appareillage, maintenu à la température et à la pression choisies, est alimenté en continu par les quantités nécessaires d'oxygène, de monooxyde d'azote et de glyoxal en solution aqueuse, acidifiée à pH inférieur à 1. La réaction est pilotée par l'analyse des gaz dégagés. En sortie de l'appareillage, la solution aqueuse d'acide glyoxylique est refroidie à la température ambiante et elle est éventuellement abandonnée à la cristallisation pour éliminer si nécessaire l'acide oxalique présent puis, si désiré, elle est soumise avantageusement à une électrodialyse, selon les techniques connues, afin d'obtenir d'une part, une solution aqueuse d'acide glyoxylique exempte de tous autres acides minéraux et/ou organiques et, d'autre part, une solution aqueuse du ou des acides minéraux utilisés.

Les exemples suivants illustrent l'invention sans toutefois la limiter.

<u>Exemple 1</u>

On alimente en continu, par l'intermédiaire d'une colonne de lavage gaz-liquide, refroidie, le premier réacteur d'une série de trois réacteurs, agités, disposés en cascade, avec une solution aqueuse de glyoxal contenant 200 g/l de glyoxal (3,446 moles/l) et 73 g/ l de chlorure d'hydrogène, avec un débit de 50 l/h, soit 172,3 moles/h de glyoxal.

Simultanément, on introduit dans le premier réacteur, d'une part de l'oxygène, de manière à maintenir dans l'appareillage une pression de 4 x $10^5$ Pa et d'autre part, 360 g/h (12 moles/h) de monooxyde d'azote. La température est maintenue à 45 ± 3°C dans le premier réacteur, à 60 ± 5°C dans le second, et à 68 ± 3°C dans le troisième. Le temps de séjour moyen de la solution réactionnelle est d'environ 70 minutes dans l'ensemble de l'appareillage. Les gaz issus des deux derniers réacteurs sont recyclés dans le premier réacteur et celui-ci est en communication avec l'extérieur par l'intermédiaire de sa colonne de lavage gaz-liquide équipée en tête

d'une soupape timbrée à 4 bars.

En sortie du troisième réacteur, on recueille 50 l/h d'une solution aqueuse d'acide glyoxylique, incolore, quasiment exempte d'acide nitrique et d'oxydes d'azote et contenant 209,5 g/l (2,83 moles/l) d'acide glyoxylique, 73 g/l (2 moles/l) de chlorure d'hydrogène, 52,8 g/l (0,58 moles/l) d'acide oxalique et 1,75 g/l (30 mmoles/l) de glyoxal. Le taux de transformation du glyoxal est de 99.1 % et la sélectivité de la réaction par rapport au glyoxal consommé est de 82,8 %.

Le bilan réactionnel par rapport au glyoxal mis en oeuvre est sensiblement quantitatif et la consommation de monooxyde d'azote est d'environ 12 moles/h et celle d'oxygène est d'environ 3200 g/h (100 moles/h).

Exemple 2

Dans un tricol de 2 litres équipé d'une agitation mécanique, d'un thermomètre plongeant, d'une ampoule à introduction, d'une arrivée d'oxygène par tube plongeant muni d'un diffuseur en verre fritté et d'un réfrigérant à reflux surmonté d'un tube à dégagement barbotant dans une solution aqueuse de soude puis dans une garde à mercure permettant d'établir dans l'appareillage une pression de 1,225 x $10^5$ Pa, on introduit :

– 1118 g d'une solution aqueuse contenant 174,12 g (3 moles) de glyoxal et 87,6 g (2,4 moles) de chlorure d'hydrogène.

Après purge soigneuse de l'appareillage avec de l'oxygène, cette solution est chauffée sous agitation à 45°C.

On introduit ensuite dans cette solution, en 15 minutes, en maintenant la température à 45°C et en réglant l'introduction d'oxygène de manière à n'avoir aucun dégagement dans la garde à mercure :

– 7,2 g (0,24 mole) de monooxyde d'azote.

L'introduction terminée, on poursuit le chauffage une heure à 45°C, puis 30 minutes à 55°C et enfin 30 minutes à 60°C sous un léger barbotage d'oxygène. On arrête alors l'arrivée d'oxygène puis on termine la réaction en maintenant le milieu réactionnel 30 minutes à 60°C.

Après refroidissement du milieu réactionnel à la température ambiante sous un léger balayage d'azote, on recueille 1173 g d'une solution aqueuse, incolore, contenant :

– 181,3 g (2,45 moles) d'acide glyocylique,
– 87,6 g (2,4 moles) d'acide chlorhydrique,
– 43,2 g (480 mmoles) d'acide oxalique anhydre,
– 3,5 g (60 mmoles) de glyoxal,
– 0,35 g (6 mmoles) d'acide nitrique.

Dans le piège à soude, on dose 1,2 g (27 mmoles) de dioxyde de carbone sous la forme de carbonate.

Le taux de transformation est de 98 %, la sélectivité est de 83,3 % en acide glyoxylique et le rendement global en acide glyoxylique est de 81,7 % de la théorie calculée, par rapport au glyoxal mis en oeuvre. La consommation en oxygène est d'environ 54 g.

Il va de soi que la présente invention n'a été décrite qu'à titre purement explicatif et nullement limitatif et que toute modification, notamment au niveau des équivalences techniques, pourra y être apportée sans sortir de son cadre.

## Revendications

1. Procédé de préparation de solutions aqueuses d'acide glyoxylique, caractérisé en ce qu'il consiste à faire réagir à un pH inférieur à 1, une solution aqueuse de glyoxal avec de l'oxygène, en présence de quantités catalytiques de monooxyde d'azote.

2. Procédé selon la revendication 1, caractérisé en ce que la solution aqueuse de glyoxal est maintenue à un pH inférieur à 1 par addition d'acide minéral fort et notamment d'acide chlorhydrique.

3. Procédé selon l'une quelconque des revendications 1 ou 2, caractérisé en ce que la proportion de monooxyde d'azote est comprise entre 75 et 150 millimoles par mole de glyoxal à oxyder.

4. Procédé selon l'une quelconque des revendications précédentes, caractérisé en ce qu'on opère sous une pression d'oxygène moléculaire comprise entre $10^5$ et 8 x $10^5$ Pa.

5. Procédé selon l'une quelconque des revendications précédentes, caractérisé en ce qu'on opère à une température comprise entre 35 et 75, et avantageusement entre 40 et 70°C.

## Patentansprüche

1. Verfahren zur Herstellung von wässerigen Glyoxylsäurelösungen, dadurch gekennzeichnet, daß man bei einem pH von niedriger als 1 eine wässerige Glyoxallösung mit Sauerstoff in Anwesenheit katalytischer Mengen Stickstoffmonoxid reagieren läßt.

2. Verfahren nach Anspruch 1, dadurch gekennzeichnet, daß die wässerige Glyoxallösung durch Zusatz einer starken Mineralsäure, insbesondere Salzsäure, bei einem pH von niedriger als 1 gehalten wird.

3. Verfahren nach Anspruch 1 oder 2, dadurch gekennzeichnet, daß die Menge an Stickstoffmonoxid 75 bis 150 Millimol pro Mol zu oxidierendem Glyoxal beträgt.

4. Verfahren nach einem der vorhergehenden Ansprüche, dadurch gekennzeichnet, daß unter einem Druck von molekularem Sauerstoff von $10^5$ bis 8 x $10^5$ Pa gearbeitet wird.

5. Verfahren nach einem der vorhergehenden Ansprüche, dadurch gekennzeichnet, daß bei einer Temperatur von 35 bis 75°C, vorteilhafterweise 40 bis 70°C, gearbeitet wird.

## Claims

1. Process for the preparation of aqueous solutions of glyoxylic acid, characterized in that it consists of reacting an aqueous solution of glyoxal with oxygen, at a pH value less than 1, in the presence of catalytic quantities of nitrogen monoxide.

2. Process according to claim 1, characterized in that the aqueous solution of glyoxal is kept at a pH value lower than 1 by the addition of a strong mineral acid and in particular hydrochloric acid.

3. Process according to either claim 1 or 2, characterized in that the proportion of nitrogen monoxide is between 75 and 150 millimoles per mole of glyoxal to be oxidized

4. Process according to any one of the preceding claims, characterized in that it is carried out under a molecular oxygen pressure of between $10^5$ and 8 x $10^5$Pa.

5. Process according to any one of the preceding claims, characterized in that it is carried out at a temperature between 35° and 75° and advantageously' between 40° and 70°C.